# EUROPÄISCHE PATENTSCHRIFT

(11) **EP 2 424 607 B1**
(45) Veröffentlichungstag und Bekanntmachung des Hinweises auf die Patenterteilung: **12.06.2019**
(21) Anmeldenummer: 10722559.1
(22) Anmeldetag: 27.04.2010
(51) Int. Cl.: A61M 25/00, A61B 5/155, A61J 1/14

(54) **TRANSFUSIONSVERWEILKATHETER, TRANSFUSIONSKANÜLEN-KIT SOWIE VERFAHREN ZUM PRÜFEN EINES TRANSFUSIONSSYSTEMS**
INDWELLING TRANSFUSION CATHETER, TRANSFUSION CANNULA KIT AND METHOD FOR TESTING A TRANSFUSION SYSTEM
SONDE À DEMEURE POUR TRANSFUSION, KIT DE CANULES POUR TRANSFUSION ET PROCÉDÉ DE CONTRÔLE DE SYSTÈME DE TRANSFUSION

(30) Priorität: 28.04.2009 DE 102009018837
(43) Veröffentlichungstag der Anmeldung: 07.03.2012
(73) Patentinhaber: Charité - Universitätsmedizin Berlin, 10117 Berlin (DE)
(72) Erfinder: NOTTER, Michael Dr., 12205 Berlin (DE)
(74) Vertreter: Bittner, Thomas L.
(86) Internationale Anmeldenummer: PCT/DE2010/000479
(87) Internationale Veröffentlichungsnummer: WO 2010/124677

(56) Entgegenhaltungen:
- EP-A2- 1 346 740
- WO-A1-03/026724
- WO-A1-2005/050497
- WO-A1-2007/128144
- WO-A1-2008/114164
- DE-A1-102004 055 989
- US-A- 4 122 947
- US-A- 5 971 972
- US-A1- 2004 193 453

## Beschreibung

Die Erfindung bezieht sich auf ein Transfusionsverweilkatheter, eine Transfusionskanülen-Kit sowie ein Verfahren zum Prüfen eines Transfusionssystems.

### Hintergrund der Erfindung

Verweilkatheter können im Rahmen einer einem Patienten zu verabreichenden Transfusion verwendet werden die in Verbindung mit einer Transfusion in Verbindung mit einer einem Patienten zu verabreichenden Transfusion verwendet werden. Andere Bezeichnungen umfassen die Begriffe peripherer Venenkatheter und periphervenöser Katheter. Bekannte Verweilkatheter, wie sie zum Beispiel in dem Dokument WO 2006/053516 A1 beschrieben sind, verfügen üblicherweise über eine Punktionskanüle, die zum Beispiel aus einem Metallmaterial wie einer Stahllegierung besteht und eine Spitze zum Einstechen aufweist. Die Punktionskanüle ist beim Setzen des Verweilkatheters in einer Nutzungsstellung in eine Verweilkanüle eingeschoben, so dass die Spitze der Punktionskanüle vorn heraussteht. Mit Hilfe der Spitze der Punktionskanüle wird der Verweilkatheter gesetzt, indem mittels der Punktionskanüle zu dem gewünschten Blutgefäß, insbesondere eine Vene, vorgestoßen wird. Nach dem Setzen des Verweilkatheters kann die Punktionskanüle entnommen werden. In Verbindung mit dem Blutgefäß verbleibt die Verweilkanüle, über die dann eine Transfusionsflüssigkeit intravenös injiziert werden kann. Demgegenüber ist eine weitere Blutentnahme über einen gesetzten Verweilkatheter häufig nicht möglich, weil hiermit verbundene Blutuntersuchungen möglicherweise durch Blutbestandteile oder Verdünnungseffekte bei vorherlaufenden Infusionen verfälscht werden können.

Deshalb ist vorgesehen, eine Blutentnahme zur Bestimmung des Blutes des Patienten in Verbindung mit dem Setzen des Verweilkatheters durchzuführen. Hierzu verfügt der Verweilkatheter über ein Reservoir, welches in einer Nutzungsstellung mit dem Ende der Punktionskanüle in Verbindung steht, so dass Blut durch die Punktionskanüle hindurch in das Reservoir fließen kann. Die so entnommene Blutprobe kann dann genutzt werden, um das Blut des Patienten zu bestimmen. Mittels statistischer Erhebungen wurde festgestellt, dass Verwechslungen von Blutprodukten mit einer Häufigkeit von etwa 1:10000 bis 1:20000 auftreten. Verwechslungen bei der Gewinnung einer Prätransfusionsblutprobe kommen in 1:3000 Fällen vor.

In Verbindung mit Bluttransfusionen wurde zur Vermeidung von Falschbehandlungen vorgeschlagen, dem Patienten ein Armband umzulegen, auf dem ein so genannter RFID-Chip angeordnet ist. RFID ist eine als solche bekannte Technologie, bei der elektronische Speicherchips genutzt werden, um Informationen zu speichern. Es sind Speicherchips bekannt, die nur auslesbar oder zusätzlich beschreibbar sind. Die Datenübertragung zwischen den Speicherchips und einem Schreib-/Lesegerät erfolgt mittels drahtloser Datenkommunikation. Ein zweiter RFID-Chip soll nun auf einer Blutkonserve angeordnet sein. Wenn eine Bluttransfusion beabsichtigt ist, wird mit einem Lesegerät die elektronische Information auf dem Armband einerseits und der Blutkonserve andererseits elektronisch ausgewertet. Auf diese Weise kann eine mit der Transfusion beauftragte Person prüfen, ob für den Patienten mit dem Armband die zur Verfügung gestellte Blutkonserve zulässig und geeignet ist. Zusätzlich wurde vorgeschlagen, dass auf dem RFID-Chip der Blutkonserve Informationen darüber gespeichert werden, bei welchen Temperaturen die Blutkonserve gelagert wurde. Zu diesem Zweck weist der RFID-Chip einen Temperatursensor auf.

Die Nutzung der RFID-Technologie als solcher im Rahmen der Arbeitsprozesse einer Blutbank ist aus dem Dokument EP 1 850 289 A1 bekannt.

In der täglichen Praxis haben sich Probleme dahingehend gezeigt, dass Armbänder der Patienten versehentlich durchschnitten werden und hierdurch verlorengehen. Derartige Situationen treten insbesondere in Verbindung mit Notfallumständen auf, in denen Patienten schnell versorgt werden müssen und die häufig durch Unübersichtlichkeit der Lage gekennzeichnet sind.

Das Dokument EP 1 346 740 A2 offenbart ein System zum Abnehmen von Blut, wobei eine Nadel mit einer Schutzvorrichtung versehen ist.

In dem Dokument WO 2007/128144 A1 ist eine Vorrichtung zum kontinuierlichen Abgeben eines medizinischen Wirkstoffs offenbart. Die Vorrichtung weist einen Datenspeicher zum Austausch von Daten auf.

Ein System zum Überprüfen von Behältern von Körperflüssigkeiten ist in dem Dokument WO 2008/114164 A1 offenbart.

Das Dokument US 2004/0193453 A1 beschreibt ein Kommunikationssystem und ein Verfahren zur Verwendung mit Infusionssystemen. Mittels des Systems können Daten zur Infusion übertragen werden WO2005/050497 beschreibt ein System und Verfahren zur Behandlung von Patienten in dem Blut entnommen, behandelt und wieder eingeführt wird. Eine besondere Kennung wird an der Blutprobe befestigt und bearbeitet und dient später zur Kontrolle, bevor die Probe wieder dem Patienten zugeführt wird.

Ein Venenverweilkatheder ist in dem Dokument DE 10 2004 055 989 A1 offenbart.

### Zusammenfassung der Erfindung

Aufgabe der Erfindung ist es, verbesserte Techniken in Verbindung mit Verweilkathetern anzugeben, die die Sicherheit in Verbindung mit der Nutzung derartiger Katheter optimieren. Insbesondere sollen fehlerhafte Transfusionen ausgeschlossen werden.

Diese Aufgabe wird erfindungsgemäß durch ein Verweilkatheter nach dem unabhängigen Anspruch 1, ein Transfusionskanülen-Kit nach dem unabhängigen Anspruch 11 sowie ein Verfahren zum Prüfen eines Transfusionssystems nach dem unabhängigen Anspruch 13 gelöst. Vorteilhafte Ausgestaltungen der Erfindung sind Gegenstand von abhängigen Unteransprüchen.

Die Erfindung umfasst nach einem Aspekt den Gedanken eines Verweilkatheters mit einer Kanüleneinrichtung und einem Reservoir, welches in einer Nutzungsstellung mit der Kanüleneinrichtung in Verbindung steht, derart, dass Blut aus der Kanüleneinrichtung in das Reservoir fließen kann, wobei das Gehäuse Gehäusekennungsmittel und das Reservoir Reservoirkennungsmittel aufweisen und die Gehäusekennungsmittel sowie die Reservoirkennungsmittel einander zugeordnete Kennungen angeben.

Nach einem weiteren Aspekt der Erfindung ist ein Transfusionskanülen-Kit mit einem Verweilkatheter geschaffen, welcher die folgenden Merkmale aufweist: Eine Kanüleneinrichtung und ein Reservoir, welches in einer Nutzungsstellung mit der Kanüleneinrichtung in Verbindung steht, derart, dass Blut aus der Kanüleneinrichtung in das Reservoir fließen kann. Weiterhin sind Kennungsmittel vorgesehen, die Gehäusekennungsmittel und Reservoirkennungsmittel umfassen, wobei die Gehäusekennungsmittel und die Reservoirkennungsmittel einander zugeordnete Kennungen angeben.

Ein anderer Aspekt der Erfindung betrifft ein Verfahren zum Prüfen eines Transfusionssystem, wobei das Verfahren die folgenden Schritte umfasst: Bereitstellen eines Verweilkatheters, bei dem ein Gehäuse mit Gehäusekennungsmitteln versehen ist, Bereitstellen einer Transfusionsflüssigkeit in einem Flüssigkeitsreservoir, welches mit Flüssigkeitskennungsmitteln versehen ist, Auswerten einer von den Gehäusekennungsmitteln angegebenen Kennung und einer von den Flüssigkeitskennungsmitteln angegebenen Kennung mit Hilfe einer Auswerteeinrichtung, indem die beiden Kennungen erfasst und verglichen werden, und Ausgeben einer Signalisierung mittels der Auswerteeinrichtung in Abhängigkeit davon, ob die Kennungen beim Vergleichen als einander zugeordnet erkannt werden oder nicht. Es ist auf diese Weise ein Verfahren zur Prozesskontrolle bei einer Transfusion geschaffen.

Bei dem vorgeschlagenen Verweilkatheter sind sowohl das Gehäuse mit der hieran gebildeten Kanüleneinrichtung als auch das Reservoir mit jeweils zugeordneten Kennungsmitteln versehen. Wenn nun das Reservoir beim Setzen des Verweilkatheters mit Blut gefüllt wird, welches durch die Kanüleneinrichtung in das Reservoir fließt, kann das so entnommene Blut, bei dem es sich um eine Prätransfusionsblutprobe handelt, in dem mit den Reservoirkennungsmitteln versehenen Reservoir zu einer Blutbestimmung genutzt werden. Die Reservoirkennungsmittel identifizieren die entnommene Blutprobe als zu dem gesetzten Verweilkatheter, nämlich dem Gehäuse mit der Kanüleneinrichtung zugehörig, was letztlich einer Zuordnung der Blutprobe zu dem Patienten mit dem Verweilkatheter entspricht. Eine aus den Reservoirkennungsmitteln abgeleitete Kennung kann dann genutzt werden, um ein neues Kennungsmittel zu erzeugen und diese zu nutzen, eine Transfusionsflüssigkeit, zum Beispiel eine Blutkonserve, mit Flüssigkeitskennungsmitteln zu kennzeichnen, zum Beispiel mittels Aufkleben der neuen Kennungsmittel auf einen Transfusionsbeutel. Auf diese Weise wird die Transfusionsflüssigkeit quasi dem Gehäuse des Verweilkatheters und somit dem Patienten mit dem Verweilkatheter zugeordnet. Die Reservoirkennungsmittel können hierbei zum Beispiel vervielfältigt werden. Oder es kann eine der Kennung aus den Reservoirkennungsmitteln abgeleitete Kennung zugeordnet werden.

Soll später dann die so gekennzeichnete Transfusionsflüssigkeit injiziert werden, kann die hiermit beauftragte Person mit Hilfe einer geeigneten Auswerteeinrichtung die Kennung an der Transfusionsflüssigkeit einerseits und die Gehäusekennungsmittel an dem Gehäuse des Verweilkatheters andererseits auswerten und vergleichen, um so festzustellen, ob die Transfusionsflüssigkeit für den Patienten, für den das Blut bestimmt und bei dem der Verweilkatheter gesetzt wurde, nutzbar ist. Die Auswerteeinrichtung ist konfiguriert, je nach Art des benutzten Kennungsmittels die zugehörige Kennung auszulesen und zu vergleichen. Es kann dann vorgesehen sein, dass der Benutzer der Auswerteeinrichtung in Abhängigkeit vom Ergebnis des Vergleichs der Kennung der Gehäusekennungsmittel einerseits und der Flüssigkeitskennungsmittel andererseits eine optische und / oder akustische Signalisierung bekommt, zum Beispiel in Form unterschiedlicher Farbsignale, um die Übereinstimmung oder Nicht-Übereinstimmung der beiden Kennungen anzuzeigen.

Mit Hilfe des vorgeschlagenen Verweilkatheters ist es ermöglicht, vom Zeitpunkt des Setzens des Verweilkatheters beim Patienten, insbesondere im Rahmen einer Notfallversorgung oder einer regulären Transfusionsversorgung, über den Prozess der Bestimmung einer hierbei entnommenen Blutprobe bis hin zu einer Transfusion über den gesetzten Verweilkatheter als geschlossenen Kreislauf zu betrachten, bei dem in den einzelnen Schritten stets eine Kennung erhalten bleibt, die vom ursprünglich gesetzten Verweilkatheter ausgeht und insoweit eindeutig dem Patienten zuzuordnen ist, bei dem der Verweilkatheter gesetzt wurde.

Die von den unterschiedlichen Kennungsmitteln, also zum Beispiel den Gehäusekennungsmitteln und dem Reservoirkennungsmitteln, zur Verfügung gestellten Kennungen können identisch sein, zum Beispiel in Form eines einheitlichen Zahlencodes. Es kann aber auch vorgesehen sein, dass die verschiedenen Kennungen eine gemeinsame Stammkennung und unterschiedliche Identifizierungskennungen enthalten, so dass anhand der Identifizierungskennung zum Beispiel erkennbar ist, ob es sich um die Kennung des Gehäuses oder des Reservoirs handelt. Wesentlich ist, dass die Kennungen einem gemeinsamen Ursprung zuordenbar sind, nämlich dem gesetzten Verweilkatheter.

Eine bevorzugte Weiterbildung der Erfindung sieht vor, dass die Gehäusekennungsmittel und / oder die Reservoirkennungsmittel ein auslesbares elektronisches Speichermedium umfassen. Bei dem elektronisch auslesbaren Speichermedium kann es sich insbesondere um einen RFDI-Chip handeln. Dieser kann in einer Ausgestaltung darüber hinaus beschreibbar ausgeführt sein. Auswerteeinrichtungen, die konfiguriert sind, RFID-Chips auszulesen und / oder zu beschreiben sind als solche in verschiedenen Ausführungen bekannt.

Bei einer zweckmäßigen Ausgestaltung der Erfindung kann vorgesehen sein, dass die Gehäusekennungsmittel und / oder die Reservoirkennungsmittel eine Strichcodekennung umfassen. Strich- oder Barcodescanner, die nutzbar sind, um Strichcodemarkierungen auszuwerten, sind als solche in unterschiedlichen Gestaltungen bekannt.

Eine vorteilhafte Ausführungsform der Erfindung sieht vor, dass die Gehäusekennungsmittel und / oder die Reservoirkennungsmittel eine alphanumerische Kennung umfassen. Die alphanumerische Kennung kann eine beliebige Anzahl von alphanumerischen Zeichen umfassen, die einander zugeordnete Kennungen angeben.

Bevorzugt sieht eine Fortbildung der Erfindung vor, dass das Reservoir mit Hilfe einer flexiblen Befestigungseinrichtung an dem Gehäuse gehalten wird. In einer Ausgestaltung handelt es sich bei der flexiblen Befestigungseinrichtung um ein flexibles Plastikband, mit dem das Reservoir an dem Gehäuse des Verweilkatheters gehalten wird. In einer Ausführungsform kann eine Sollbruchstelle im Bereich der flexiblen Befestigungseinrichtung vorgesehen sein, was das Lösen des Reservoirs von dem Gehäuse des Verweilkatheters erleichtert. Alternativ kann das Abtrennen des Reservoirs vom Gehäuse auch mittels Durchschneiden der flexiblen Befestigungseinrichtung vom Benutzer vorgenommen werden. In den verschiedenen Ausgestaltungen wird das Reservoir mit Hilfe der flexiblen Befestigung an dem Gehäuse so gehalten, dass es einerseits in die Nutzungsstellung gebracht werden kann, in welcher die wenigstens teilweise Befüllung des Reservoirs mit Blut beim Setzen des Verweilkatheters stattfindet. Das Reservoir kann andererseits aber auch aus dieser Nutzungsstellung gelöst werden und hängt dann im Wesentlichen frei an dem Gehäuse. Bei einer Weiterbildung kann vorgesehen sein, dass an dem Gehäuse eine Aufnahme, insbesondere eine Steckaufnahme gebildet ist, in die das Reservoir nach und / oder vor dem Befüllen mit Blut eingesteckt werden kann. Auch ist die flexible Befestigung in einer Fortbildung mit einer ausreichenden Länge versehen, so dass das Reservoir seitlich der gesetzten Verweilkanüle mittels eines Pflasters auf der Haut des Patienten fixiert werden kann, um es zum Beispiel nach dem Transport eines Notfallpatienten von der Unfallstelle zum Krankenhaus dort zu lösen und beispielsweise einer Blutbestimmung zuzuführen.

Bei einer vorteilhaften Ausgestaltung der Erfindung kann vorgesehen sein, dass das Reservoir als ein passiv füllendes Reservoir gebildet ist. Bei dem passiv füllenden Reservoir ist eine Öffnung vorgesehen, durch die Luft beim Befüllen des Reservoirs mit Blut entweichen kann, so dass das Blut in das Reservoir strömen kann. Bevorzugt ist die Öffnung mit einem Filter bedeckt. Alternativ zum passiv füllenden Reservoir kann in einer Ausgestaltung ein aktiv füllendes Reservoir vorgesehen sein, bei dem die Befüllung mit Unterstützung eines Vakuums stattfindet, das im Inneren des Reservoirs ausgebildet ist.

Eine Weiterbildung der Erfindung kann vorsehen, dass die Gehäusekennungsmittel an dem Gehäuse und / oder die Reservoirkennungsmittel an dem Reservoir lösbar befestigt sind. Eine lösbare Befestigung kann beispielsweise mittels einer haftenden Verbindung ausgeführt sein.

Eine bevorzugte Weiterbildung der Erfindung sieht vor, dass die Gehäusekennungsmittel und / oder die Reservoirkennungsmittel auf einem Träger angeordnet sind, welcher lösbar an dem Gehäuse / Reservoir angeordnet ist. In einer Ausführungsform ist an dem Träger ein Befestigungsmittel gebildet, mit dem der Träger und somit das Kennungsmittel an dem Gehäuse und / oder dem Reservoir befestigbar ist. Beispielsweise kann das Befestigungsmittel mit einem so genannten Klippanschluss versehen sein.

Bei einer zweckmäßigen Ausgestaltung der Erfindung kann vorgesehen sein, dass an dem Gehäuse und / oder an dem Reservoir weitere Kennungsmittel für eine Nutzung getrennt von dem Gehäuse / Reservoir bereitgestellt sind, die ebenfalls eine zugeordnete Kennung angeben. Bei dieser Ausführungsform ist an dem Gehäuse und / oder an dem Reservoir eine Art Magazin mit Kennungsmitteln gebildet, die dazu genutzt werden können, andere Gegenstände in gleicher Art und Weise mit einer Kennung zu versehen, zum Beispiel einen Transfusionsbeutel. In einer Ausführungsform sind die weiteren Kennungsmittel in Form eines oder mehrerer Klebeetiketten oder Klebechips ausgeführt. Auch kann vorgesehen sein, dass mehrere Kennungsmittel untereinander über eine Sollbruch- oder Sollreißstelle verbunden sind, so dass sie vom Benutzer Stück für Stück abgetrennt und verwendet werden können. Bevorzugt verfügen die weiteren Kennungsmittel über eine Haftfläche, die es ermöglicht, dass Kennungsmittel auf einem gewünschten Gegenstand haftend anzubringen.

Eine Fortbildung der Erfindung kann vorsehen, dass die Kanüleneinrichtung mit einer Punktionskanüle zum Punktieren und einer an dem Gehäuse gebildeten Verweilkanüle ausgeführt ist, durch welche hindurch die Punktionskanüle in einer eingeführten Stellung verläuft. Bei dieser Ausführungsform dient die Punktionskanüle zum Punktieren beim Setzen des Verweilkatheters. Zu diesem Zweck ist an der Punktionskanüle eine Stechspitze vorgesehen. Hierbei wird die Punktionskanüle dann auch von Blut durchströmt, welches in das Reservoir fließt, wenn dieses in der Nutzungsstellung ist. Das Reservoir ist in der Nutzungsstellung in Fließverbindung mit der Punktionskanüle, indem das Reservoir zum Beispiel auf ein Endstück der Punktionskanüle aufgesteckt ist, beispielsweise über einen sogenannten Luer-Anschluss. Anschließend können Reservoir und Punktionskanüle gelöst werden, worauf die Verweilkanüle gesetzt bleibt. Alternativ ist der Kanüleneinrichtung mit nur einer Kanüle gebildet, die sowohl für das Punktieren beim Setzen des Katheters als auch zum Verweilen im Körper des Patienten für eine spätere Transfusion konfiguriert ist.

Die in Verbindung mit dem Verweilkatheter vorangehend erläuterten Ausführungsformen können für das Transfusionskanülen-Kit und bei dem Verfahren zum Prüfen eines Transfusionssystem einzeln oder in Kombination in analoger Weise vorgesehen sein.

### Beschreibung bevorzugter Ausführungsbeispiele der Erfindung

Die Erfindung wird im Folgenden anhand von Ausführungsbeispielen unter Bezugnahme auf Figuren einer Zeichnung näher erläutert. Hierbei zeigen:
- Fig. 1: eine schematische Darstellung zur Erläuterung der Nutzung von Kennungsmitteln in Verbindung mit einem Verweilkatheter und
- Fig. 2: eine schematische Darstellung einer lösbaren Trägereinrichtung für ein Kennungsmittel an einem Verweilkatheter.

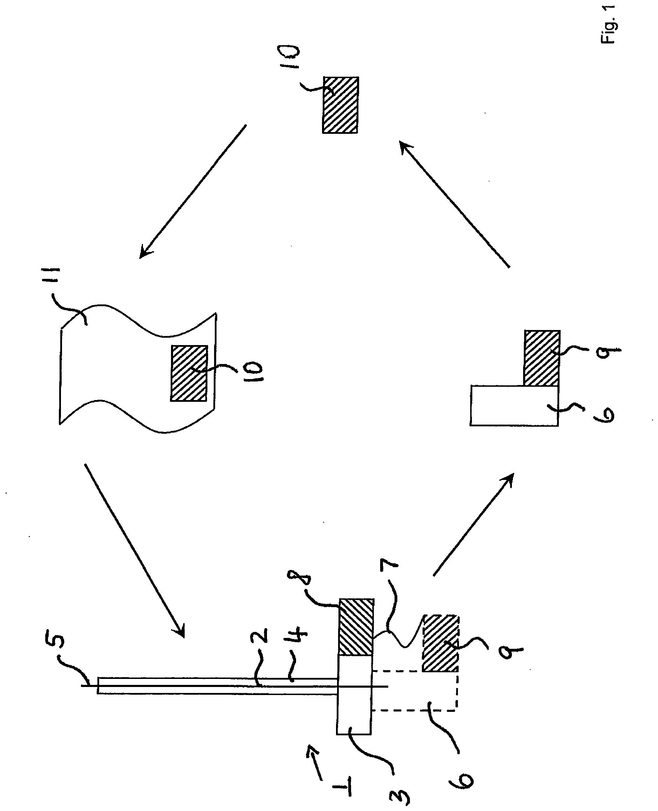

Fig. 1 zeigt eine schematische Darstellung zur Erläuterung eines Verweilkatheter- bzw. Transfusionsverweilkathetersystems. Bei einem Verweilkatheter 1 ist eine Punktionskanüle 2 in einem Gehäuse 3 eine Verweilkanüle 4 durchgreifend angeordnet, so dass eine Spitze 5 der Punktionskanüle 2 aus der Verweilkanüle 4 heraussteht. Beim Setzten des Verweilkatheters 1 wird die Haut des Patienten mittels der Spitze 5 der Punktionskanüle 2 aufgestochen. Gelangt die Spitzt 5 der Punktionskanüle 2 zu dem Blutgefäß, insbesondere der Vene, kann durch die Punktionskanüle 2 Blut in einem Reservoir 6 gelangen, welches an dem Gehäuse 3 des Verweilkatheters 1 angeordnet ist. Das Reservoir 6 ist mit Hilfe einer flexiblen Befestigung 7, zum Beispiel einem Plastikband, an dem Gehäuse 3 des Verweilkatheters befestigt.

Gemäß Fig. 1 sind das Gehäuse 3 des Verweilkatheters 1 mit Gehäusekennungsmitteln 8 sowie das Reservoir 6 mit Reservoirkennungsmitteln 9 versehen. Die mit den beiden Kennungsmitteln 8, 9 angegebenen Kennungen sind einander zugeordnet.

Unmittelbar nach dem Befüllen oder zu einem späteren Zeitpunkt kann das Reservoir 7 gemäß Fig. 1 von dem Verweilkatheter 1 abgetrennt werden, einschließlich der Reservoirkennungsmittel 9, und einer Blutbestimmung des entnommenen Blutes zugeführt werden. Die Reservoirkennungsmittel 9 können dann weiterhin dupliziert werden, so dass neue Kennungsmittel 10 hergestellt werden, die anschließend auf einen Beutel 11 mit einer Transfusionsflüssigkeit aufgeklebt werden können. Wenn dann dem Patienten, welchem der Verweilkatheter 1 gesetzt wurde, die Transfusionsflüssigkeit aus dem Transfusionsbeutel 11 injiziert werden soll, prüft die hiermit beauftragte Person mittels Auswerten der Gehäusekennungsmittel 8 sowie der neuen Kennungsmittel 10 die Eignung und Zulässigkeit der Transfusionsflüssigkeit.

Das Erzeugen der neuen Kennungsmittel 10 kann dadurch ausgeführt werden, dass die Reservoirkennungsmittel 9 gelesen werden, zum Beispiel mittels eines optischen Scanners, und anschließend die neuen Kennungsmittel 10 gedruckt werden, was zum Beispiel in Verbindung mit einer Kennung in Form eines Strichcodes oder einer alphanumerischen Zeichenkette möglich ist. Werden Kennungsmittel in Form eines elektronischen Speichermediums verwendet, wird eine neues Speichermittel entsprechend beschrieben mit Informationsdaten, die es beim Auswerten später ermöglichen, die Zuordnung zu dem Patienten mit dem Verweilkatheter zu treffen. Es ist so die Aktionskette vom Setzen des Verweilkatheters 1 bis zum Verabreichen einer Transfusion geschlossen.

Bei der in Fig. 1 dargestellten Ausführungsform ist der Verweilkatheter 1 mit einer Punktionskanüle 2 sowie der Verweilkanüle 4 gebildet. Alternativ kann vorgesehen sein, dass der Verweilkatheter nur über eine Kanüle verfügt, die sowohl zum Setzen des Katheters als auch zum Verbleib für eine spätere Transfusion geeignet ist.

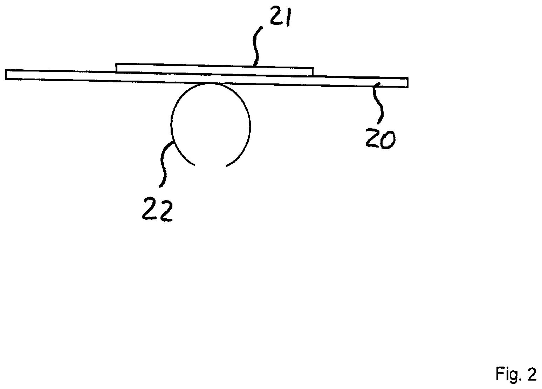

Fig. 2 zeigt eine mögliche Ausführungsform für ein Befestigungsmittel zum lösbaren Anbringen eines Kennungsmittels an dem Verweilkatheter 1 in Fig. 1. Auf einem Träger 20 ist ein Kennungsmittel 21 aufgebracht, zum Beispiel aufgeklebt. An dem Verweilkatheter 1 kann der Träger 20 nun mit Hilfe der Befestigungsmittel 22 lösbar angebracht werden, bei dem es sich um eine Art Klippbefestigung handelt.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungen von Bedeutung sein.

## Patentansprüche

1. Transfusionssystem mit
- einem Verweilkatheter, mit
- einem Gehäuse (3) mit einer Kanüleneinrichtung (2, 4), welches Gehäusekennungsmittel (8) aufweist, und
- einem Reservoir (6), welches in einer Nutzungsstellung mit der Kanüleneinrichtung (2, 4) in Verbindung steht, derart, dass Blut aus die Kanüleneinrichtung (2, 4) in das Reservoir (6) fließen kann, wobei das Reservoir (6) Reservoirkennungsmittel (9) aufweist und wobei die Reservoirkennungsmittel (9) sowie die Gehäusekennungsmittel (8) einander zugeordnete Kennungen angeben, und
- einem Flüssigkeitsreservoir (11), welches mit Flüssigkeitskennungsmitteln (10) versehen ist, wobei die Flüssigkeitskennungsmittel (10) von den Reservoirkennungsmitteln abgeleitet sind.

2. Transfusionssystem nach Anspruch 1, **dadurch gekennzeichnet, dass** die Gehäusekennungsmittel (8) und / oder die Reservoirkennungsmittel (9) ein auslesbares elektronisches Speichermedium umfassen.

3. Transfusionssystem nach Anspruch 1 oder 2, **dadurch gekennzeichnet, dass** die Gehäusekennungsmittel (8) und / oder die Reservoirkennungsmittel (9) eine Strichcodekennung umfassen.

4. Transfusionssystem nach mindestens einem der vorangehenden Ansprüche, **dadurch gekennzeichnet, dass** die Gehäusekennungsmittel (8) und / oder die Reservoirkennungsmittel (9) eine alphanumerische Kennung umfassen.

5. Transfusionssystem nach mindestens einem der vorangehenden Ansprüche, **dadurch gekennzeichnet, dass** das Reservoir (6) mit Hilfe einer flexiblen Befestigungseinrichtung (7) an dem Gehäuse (3) gehalten wird.

6. Transfusionssystem nach mindestens einem der vorangehenden Ansprüche, **dadurch gekennzeichnet, dass** das Reservoir (6) als ein passiv füllendes Reservoir gebildet ist.

7. Transfusionssystem nach mindestens einem der vorangehenden Ansprüche, **dadurch gekennzeichnet, dass** die Gehäusekennungsmittel (8) an dem Gehäuse (3) und / oder die Reservoirkennungsmittel (9) an dem Reservoir (6) lösbar befestigt sind.

8. Transfusionssystem nach Anspruch 7, **dadurch gekennzeichnet, dass** die Gehäusekennungsmittel (8) und / oder die Reservoirkennungsmittel (9) auf einem Träger angeordnet sind, welcher lösbar an dem Gehäuse (3) / Reservoir (6) angeordnet ist.

9. Transfusionssystem nach mindestens einem der vorangehenden Ansprüche, **dadurch gekennzeichnet, dass** an dem Gehäuse (3) und / oder an dem Reservoir (6) weitere Kennungsmittel für eine Nutzung getrennt von dem Gehäuse (3) / Reservoir (6) bereitgestellt sind, die ebenfalls eine zugeordnete Kennung angeben.

10. Transfusionssystem nach mindestens einem der vorangehenden Ansprüche, **dadurch gekennzeichnet, dass** die Kanüleneinrichtung (2, 4) mit einer Punktionskanüle (2) zum Punktieren und einer an dem Gehäuse gebildeten Verweilkanüle (4) ausgeführt ist, durch welche hindurch die Punktionskanüle (2) in einer eingeführten Stellung verläuft.

11. Verfahren zum Prüfen eines Transfusionssystems, wobei das Verfahren die folgenden Schritte umfasst:
- Bereitstellen eines Verweilkatheters (1), aufweisend ein Gehäuse (3) mit einer Kanüleneinrichtung (2, 4) und einem Reservoir (6), wobei das Gehäuse (3) mit Gehäusekennungsmitteln (8) versehen ist, das Reservoir (6) mit Reservoirkennungsmitteln (9) versehen ist und die Gehäusekennungsmittel (8) sowie die Reservoirkennungsmittel (9) einander zugeordnete Kennungen angeben,
- Füllen des Reservoirs (6) mit Blut eines Patienten,
- Entfernen des Reservoirs (6) mit den Reservoirkennungsmitteln (9) von dem Verweilkathether (1),
- Auswerten der Reservoirkennungsmittel (9),
- Erzeugen von Flüssigkeitskennungsmitteln (10) mit einer aus der Auswertung der Reservoirkennungsmittel (9) abgeleiteten Flüssigkeitskennung,
- Bereitstellen einer Transfusionsflüssigkeit in einem Flüssigkeitsreservoir (11), welches mit den Flüssigkeitskennungsmitteln (10) versehen ist,
- Auswerten einer von den Gehäusekennungsmitteln (8) angegebenen Kennung und einer von den Flüssigkeitskennungsmitteln (10) angegebenen Kennung mit Hilfe einer Auswerteeinrichtung, indem die beiden Kennungen erfasst und verglichen werden, und
- Ausgeben einer Signalisierung mittels der Auswerteeinrichtung in Abhängigkeit davon, ob die Kennungen beim Vergleichen als einander zugeordnet erkannt werden oder nicht.

## Claims

1. Transfusion system with
- an indwelling catheter with
- a casing (3) with a cannula device (2, 4) which has casing identification means (8) and
- a reservoir (6) which in a usage position is in connection with the cannula device (2, 4) in such a way that blood can flow out of the cannula device (2, 4) into the reservoir (6), wherein the reservoir (6) has reservoir identification means (9) and wherein the reservoir identification means (9) and the casing identification means (8) indicate identifications assigned to each other, and
- a fluid reservoir (11) which is provided with fluid identification means (10), wherein the fluid identification means (10) are derived from the reservoir identification means.

2. Transfusion system according to claim 1 **characterised in that** the casing identification means (8) and/or the reservoir identification means (9) comprise a readable electronic storage medium.

3. Transfusion system according to claim 1 or 2, **characterised in that** the casing identification means (8) and/or the reservoir identification means (9) comprise a bar code identification.

4. Transfusion system according to at least one of the preceding claims, **characterised in that** the casing identification means (8) and/or the reservoir identification means (9) comprise an alphanumeric identification.

5. Transfusion system according to at least one of the preceding claims, **characterised in that** the reservoir (6) is held on the casing (3) by means of a flexible fastening device (7).

6. Transfusion system according to at least one of the preceding claims, **characterised in that** the reservoir (6) is designed as a passively filling reservoir.

7. Transfusion system according to at least one of the preceding claims, **characterised in that** the casing identification means (8) are detachably fastened to the casing (3) and/or the reservoir identification means (9) are detachably fastened to the reservoir (6).

8. Transfusion system according to claim 7, **characterised in that** the casing identification means (8) and/or the reservoir identification means (9) are arranged on a carrier which is detachably arranged on the casing (3)/reservoir (6).

9. Transfusion system according to at least one of the preceding claims, **characterised in that** on the casing (3) and/or on the reservoir (6) further identification means for a use separate from the casing (3)/reservoir (6) are provided which also indicate an assigned identification.

10. Transfusion system according to any one of the preceding claims, **characterised in that** the cannula device (2, 4) is designed with a puncture cannula (2) for puncturing and an indwelling cannula (4) formed on the casing, through which the puncture cannula (2) extends in an inserted position.

11. Method of testing a transfusion system, wherein the method comprises the following steps:
- providing an indwelling catheter (1), comprising a casing (3) with a cannula device (2, 4) and a reservoir (6), wherein the casing (3) is provided with casing identification means (8), the reservoir (6) is provided with reservoir identification means (9), and the casing identification means (8) and the reservoir identification means (9) indicate identifications assigned to each other,
- filling the reservoir (6) with blood of a patient,
- removing the reservoir (6) with the reservoir identification means (9) from the indwelling catheter (1),
- evaluating the reservoir identification means (9),
- generating fluid identification means (10) with a fluid identification derived from the evaluation of the reservoir identification means (9),
- providing a transfusion fluid in a fluid reservoir (11) which is provided with the fluid identification means (10),
- evaluating an identification indicated by the casing identification means (8) and an identification indicated by the fluid identification means (10) by way of an evaluation device, wherein both identifications are recorded and compared, and
- outputting a signal by means of the evaluation device depending on whether in the comparison the identifications are recognised as being assigned to each other or not.

## Revendications

1. Système de transfusion, avec
- un cathéter à demeure, avec
- un boîtier (3) doté d'un dispositif de canule (2, 4) présentant des moyens d'identification de boîtier (8), et
- un réservoir (6) qui est en liaison dans une position d'utilisation avec le dispositif de canule (2, 4) de manière à ce que du sang puisse s'écouler depuis le dispositif de canule (2, 4) dans le réservoir (6), le réservoir (6) présentant des moyens d'identification de réservoir, et les moyens d'identification de réservoir (9) et les moyens d'identification de boîtier (8) mentionnant des identifiants associés les uns aux autres, et
- un réservoir à liquide (11) qui est pourvu de moyens d'identification de liquide (10), les moyens d'identification de liquide (10) étant dérivés des moyens d'identification de réservoir.

2. Système de transfusion selon la revendication 1, **caractérisé en ce que** les moyens d'identification de boîtier (8) et/ou les moyens d'identification de réservoir (9) comprennent un support de mémoire électronique consultable.

3. Système de transfusion selon la revendication 1 ou 2, **caractérisé en ce que** les moyens d'identification de boîtier (8) et/ou les moyens d'identification de réservoir (9) comprennent un identifiant à code barre.

4. Système de transfusion selon une des revendications précédentes, **caractérisé en ce que** les moyens d'identification de boîtier (8) et/ou les moyens d'identification de réservoirs (9) comprennent un identifiant alphanumérique.

5. Système de transfusion selon une des revendications précédentes, **caractérisé en ce que** le réservoir (6) est maintenu au niveau du boîtier (3) au moyen d'un dispositif de fixation flexible (7).

6. Système de transfusion selon une des revendications précédentes, **caractérisé en ce que** le réservoir (6) est réalisé sous forme d'un réservoir se remplissant passivement.

7. Système de transfusion selon une des revendications précédentes, **caractérisé en ce que** les moyens d'identification de boîtier (8) sont fixés de manière amovible au boîtier (3) et/ou les moyens d'identification de réservoir (9) au réservoir (6).

8. Système de transfusion selon la revendication 7, **caractérisé en ce que** les moyens d'identification de boîtier (8) et/ou les moyens d'identification de réservoir (9) sont disposés sur un support qui est disposé de manière amovible au niveau du boîtier (3)/réservoir (6).

9. Système de transfusion selon une des revendications précédentes, **caractérisé en ce que**, au niveau du boîtier (3) et/ou du réservoir (6), il est prévu d'autres moyens d'identification pour une utilisation séparément du boîtier (3)/réservoir (6) et qui mentionnent également un identifiant associer.

10. Système de transfusion selon une des revendications précédentes, **caractérisé en ce que** le dispositif de canule (2, 4) est réalisé avec une canule de piquage (2) pour le piquage et une canule à demeure (4) constituée au niveau du boîtier et à travers laquelle la canule de piquage (2) passe en position introduite.

11. Procédé de contrôle d'un système de transfusion, ce procédé comprenant les étapes suivantes :
- pourvoyant d'un cathéter à demeure (1) comprenant un boîtier (3) doté d'un dispositif de canule (2, 4) et un réservoir (6), le boîtier (3) étant pourvu de moyens d'identification de boîtier (8), le réservoir (6) étant pourvu de moyens d'identification de réservoir (9), et les moyens d'identification de boîtier (8) et les moyens d'identification de réservoir (9) mentionnant des identifiants associés les uns aux autres, et
- remplissage du réservoir (6) avec du sang d'un patient,
- retrait du réservoir (6) avec les moyens d'identification de réservoir (9) du cathéter à demeure (1),
- exploitation des moyens d'identification de réservoir (9),
- génération de moyens d'identification de liquide (10) avec un identifiant de liquide dérivé de l'exploitation des moyens d'identification de réservoir (9),
- pourvoyant d'un liquide de transfusion dans un réservoir à liquide (11) qui est pourvu des moyens d'identification de liquide (10),
- exploitation d'un identifiant mentionné par les moyens d'identification de boîtier (8) et d'un identifiant mentionner par les moyens d'identification de liquide (10) à l'aide d'un dispositif d'exploitation dans lequel les deux identifiants sont enregistrés et comparés, et
- émission d'une signalisation au moyen du dispositif d'exploitation au moyen du dispositif d'exploitation selon que les identifiants sont ou non détectés comme étant associés l'un à l'autre lors de la comparaison.
